# EUROPEAN PATENT SPECIFICATION

(11) **EP 1 391 505 B1**
(45) Date of publication and mention of the grant of the patent: **28.01.2009**
(21) Application number: 02720586.3
(22) Date of filing: 24.04.2002
(51) Int. Cl.: C12N 5/08, C12N 15/09, G01N 33/50, G01N 33/15, A61K 35/39, A61P 1/18, C12Q 1/68, C12Q 1/02

(54) **STEM CELLS AND METHOD OF SEPARATING THE SAME**
STAMMZELLEN UND VERFAHREN ZU DEREN TRENNUNG
CELLULES SOUCHES ET PROCEDE D'EXTRACTION DE CES CELLULES

(30) Priority: 24.04.2001 JP 2001126315
(43) Date of publication of application: 25.02.2004
(73) Proprietor: Ajinomoto Co., Inc., Tokyo 104-8315 (JP)
(72) Inventor: TANIGUCHI, Hideki, Ushiku-shi, Ibaraki 300-1217 (JP); SUZUKI, Atsushi, Kiryu-shi, Gunma 376-0041 (JP)
(74) Representative: HOFFMANN EITLE
(86) International application number: PCT/JP2002/004084
(87) International publication number: WO 2002/088335

(56) References cited:
- WO-A-00/47720
- WO-A-97/17086
- WO-A1-97/15310
- RAMIYA VIJAYAKUMAR K ET AL: "Reversal of insulin-dependent diabetes using islets generated in vitro from pancreatic stem cells" NATURE MEDICINE, NATURE AMERICA, NEW YORK, US, vol. 6, no. 3, March 2000 (2000-03), pages 278-282, XP000864764 ISSN: 1078-8956
- PESHAVARIA M ET AL: "Manipulation of pancreatic stem cells for cell replacement therapy." DIABETES TECHNOLOGY & THERAPEUTICS. 2000 AUTUMN, vol. 2, no. 3, October 2000 (2000-10), pages 453-460, XP009034401 ISSN: 1520-9156
- SUZUKI ATSUSHI ET AL: "Establishment of clonal colony-forming assay system for pancreatic stem/progenitor cells" CELL TRANSPLANTATION, vol. 11, no. 5, 2002, pages 451-453, XP0009029522 & ISSN: 0963-6897
- SUZUKI ATSUSHI ET AL: "Clonal identification and characterization of self-renewing pluripotent stem cells in the developing liver" JOURNAL OF CELL BIOLOGY, vol. 156, no. 1, 7 January 2002 (2002-01-07), pages 173-184, XP002277322 & ISSN: 0021-9525
- BONNER-WEIR S ET AL: "PANCREATIC STEM CELLS" PROCEEDINGS OF THE SYMPOSIUM ON APPLIED COMPUTING, 5-6 APRIL 90, FAYETTEVILLE, LOS ALAMITOS, CA, US, vol. 197, no. 4, July 2002 (2002-07), pages 519-526, XP008019338
- BOUWENS LUC ET AL.: 'Islet morphogenesis and stem cell markers in rat pancreas.' THE JOURNAL OF HISTOCHEMISTRY AND CYTOCHEMISTRY vol. 44, no. 9, 1996, pages 947 - 951, XP002955658
- ZULEWSKI HENRYK ET AL.: 'Multipotential nestin-positive stem cells isolated from adult pancreatic islets differentiate ex vino into pancreatic endocrine, exocrine and hepatic phenotypes' DIABETES vol. 50, no. 3, March 2001, pages 521 - 533, XP002955659
- HUNZIKER ERIC ET AL.: 'Nestin-expressing cells in the pancreatic islets of langerhans' BIOCHEMNICAL AND BIOPHYSICAL RESEARCH COMMUNICATIONS vol. 271, no. 1, 2000, pages 116 - 119, XP002938450

## Description

### Technical Field

The present invention relates to a method for separating pancreatic stem cell of a mammal and a identification method thereof.

### Background Art

A method of separating a hepatic progenitor cell from the liver of a fetal mouse using [c-Kit⁻, CD49f⁺, CD29⁺, CD45⁻, TER119⁻] as a marker (Hepatology 32(6), 1230-1239 (2000)) and a method of separating a hepatic stem cell using [c-Met⁺, CD49f⁺/low, c-Kit⁻, CD45⁻, TER119⁻] as a marker (Hideki Taniguchi: Records of the 117th Japanese Association of Medical Sciences Symposium, Stem cell and cell therapy, 80-89 (2001)) have been reported Zulewski et al., Diabetes, Vol. 50, No. 3, March 2001, pages 521-533, disclose multipotential nestin-positive stem cells isolated from adult pancreatic islets, which differentiate in vivo into pancreatic endocrine, exocrine and hepatic phenotypes. It has been considered that an organ has a unique stem cell thereof, but the presence of a marker common to the stem cells has not been expected. In particular, a method of separation or identification of a pancreatic stem cell that differentiate variously and exerts wide-ranging physiological functions has not been established at present.

### Disclosure of the Invention

It is an object of the present invention to provide a method of separating or identifying a pancreatic stem cell.

The present inventors have conducted intensive studies in an attempt to achieve the above-mentioned object and found an expression pattern of a marker of [c-Met⁺, c-Kit⁻, CD45⁻, TER119⁻] common to pancreatic stem cells, and succeeded in preferably separating and identifying a pancreatic stem cell utilizing the characteristics. Further detailed studies have resulted in the finding that pancreatic stem cells can be preferably further separated and identified, using the expression pattern of a marker [c-Met⁺, c-Kit⁻, CD45⁻, TER119⁻, Flk-1⁻] as an index, which resulted in the completion of the present invention. Accordingly, the present invention provides the following.
(1) A method of separating a pancreatic stem cell from the pancreas of a mammal using substances having specific affinity for a marker protein selected from the group consisting of c-Met, c-Kit, CD45 and TER119 or genes encoding the same.
(2) A method of separating a pancreatic stem cell from the pancreas of a mammal using substances having specific affinity for a marker protein selected from the group consisting of c-Met, c-Kit, CD45, TER119 and Flk-1 or genes encoding the same.
(3) The method of the above-mentioned (1) or (2), wherein the substance having specific affinity is an antibody against the marker protein.
(4) A method of identifying a pancreatic stem cell of a mammal using substances having specific affinity for a marker protein selected from the group consisting of c-Met, c-Kit, CD45 and TER119 or genes encoding the same.
(5) A method of identifying a pancreatic stem cell of a mammal using substances having specific affinity for a marker protein selected from the group consisting of c-Met, c-Kit, CD45, TER119 and Flk-1 or genes encoding the same.
(6) The method of the above-mentioned (4) or (5), wherein the substance having specific affinity is an antibody against the marker protein.

A method of separating a pancreatic stem cell from the pancreas of a mammal, which comprises a step of analyzing the expression state of two or more marker proteins selected from the group consisting of c-Met, c-Kit, CD45 and TER119 or a gene encoding the same is also described.

A method of separating a pancreatic stem cell from the pancreas of a mammal, which comprises a step of analyzing the expression state of two or more marker proteins selected from the group consisting of c-Met, c-Kit, CD45, TER119 and Flk-1 or a gene encoding the same is also described.

A method of identifying a pancreatic stem cell from the pancreas of a mammal, which comprises a step of analyzing the expression state of two or more marker proteins selected from the group consisting of c-Met, c-Kit, CD45 and TER119 or a gene encoding the same is described as well.

A method of identifying a pancreatic stem cell from the pancreas of a mammal, which comprises a step of analyzing the expression state of two or more marker proteins selected from the group consisting of c-Met, c-Kit, CD45, TER119 and Flk-1 or a gene encoding the same is described.

A pancreatic stem cell that can be separated from the pancreas of a mammal by the method described in any of the above-mentioned (1), (2), (7) and (8) is disclosed.

The pancreatic stem cell as above-mentioned, which shows four markers of c-Met, c-Kit, CD45 and TER119 in a pattern of c-Met⁺, c-Kit⁻, CD45⁻, TER119⁻ is disclosed, too.

The pancreatic stem cell as above-mentioned, which shows five markers of c-Met, c-Kit, CD45, TER119 and Flk-1 in a pattern of c-Met⁺, c-Kit⁻, CD45⁻, TER119⁻, Flk-1⁻ is disclosed. An agent for the prophylaxis or treatment of a pancreatic hypofunctional disease, which comprises the pancreatic stem cell as above-mentioned, or a cell differentiated from the stem cell is mentioned.

An agent for the prophylaxis or treatment of a hypofunctional disease of the liver· bile duct or the stomach intestine, which comprises the pancreatic stem cell as above-mentioned, or a cell differentiated from the stem cell is disclosed.

A method of screening a substance that induces differentiation of a pancreatic stem cell of a mammal, which comprises the following steps:
(i) a step of reacting a pancreatic stem cell with a test substance, and
(ii) a step of determining the expression of a pancreatic marker in the cell after the reaction is described.

A method of screening a substance that induces differentiation into liver bile duct or stomach intestine of a mammal, which comprises the following steps:
(i) a step of reacting a pancreatic stem cell as above-mentioned with a test substance, and
(ii) a step of determining the expression of a liver· bile duct or stomach - intestine marker in the cell after the reaction is disclosed. A method of screening a substance that regulates a pancreatic function of a mammal, which comprises the following steps is described.
   (i) a step of reacting a pancreatic stem cell or a cell differentiated from the stem cell with a test substance, and
   (ii) a step of determining the expression of a pancreatic marker in the cell after the reaction.

A method of screening a substance that regulates the function of liver- bile duct or stomach intestine of a mammal, which comprises the following steps is mentioned:
(i) a step of reacting a pancreatic stem cell as above-mentioned or a cell differentiated from the stem cell with a test substance, and
(ii) a step of determining the expression of a liver· bile duct or stomach - intestine marker in the cell after the reaction.

### Detailed Description of the Invention

The present invention provides a method of separating or identifying a pancreatic stem cell from the pancreas of a mammal, using a substance having specific affinity for 4 or 5 marker proteins selected from the group consisting of c-Met, c-Kit, CD45, Flk-1 and TER119, or genes encoding same.

In the present specification, the "mammal" is concretely exemplified by human, bovine, horse, dog, guinea pig, mouse, rat and the like.

The c-Met protein is a protooncogene product, and functions as a hepatocyte growth factor receptor (HGF receptor) having tyrosine kinase activity. The relationship between the expression thereof and cancers in stomach, bile duct and the like has been reported. The c-Kit protein is also a similar protooncogene product, which is a receptor tyrosine kinase having a similar structure to that of a receptor of a macrophage colony-stimulating factor or a platelet-derived growth factor, and which has been reported to be involved in a certain kind of tumors. The CD45 protein is a kind of leukocyte common antigen, and is known to be expressed in all the hematopoietic cells except erythrocyte, platelet and progenitor cells thereof. The TER119 protein is known to be a cell surface marker effective for selecting erythroid cells. The Flk-1 protein is known to be an effective cell surface marker for selecting a vascular endothelial cell. The present invention is based on a new finding that these proteins and genes encoding them show a unique expression pattern in a pancreatic stem cell. In the present specification, unless particularly specified, by the "marker" is meant each member of the group consisting of a series of proteins showing such expression pattern unique to a pancreatic stem cell and genes encoding them. Such marker protein may have a different amino acid sequence depending on the kind of mammal and the like. In the present invention, as long as the expression pattern in the pancreatic stem cell is the same, such protein can be also used as a marker protein and is within the scope of the present invention. To be precise, a homologue having not less than 40% of homology in the amino acid sequence of each protein can be used as a marker protein in the present invention, namely, as c-Met, c-Kit, CD45, TER119 or Flk-1. The present invention comprises a step of analyzing the expression of such marker protein and/or a gene encoding the same, using a substance having specific affinity for each marker protein or a gene encoding the same.

In the present specification, the term "using" is not particularly limited as regards its method. Specifically, when a substance having specific affinity for, for example, a marker protein, is used, a method utilizing an antigen-antibody reaction with the antibody of said marker protein can be mentioned, and when a substance having specific affinity for a gene encoding a marker protein is used, a method utilizing a hybridization reaction can be mentioned (procedure is to be described in detail later).

As a substance having specific affinity for a marker protein, for example, an antibody having specific affinity for the protein, and a fragment thereof can be mentioned, where the specific affinity refers to an ability to specifically recognize a protein by an antigen-antibody reaction and to bind therewith. The antibody and a fragment thereof are not particularly limited as long as they can bind specifically with the protein, and may be a polyclonal antibody, a monoclonal antibody or a functional fragment thereof. These antibodies and their functional fragments can be produced according to a method generally employed in this field. For example, when a polyclonal antibody is used, a method wherein a protein is injected subcutaneously to the back of or intraperitoneally, intravenously and the like to an animal such as mouse and rabbit for immunization, and after awaiting an increase in the antibody titer, antiserum is harvested, can be mentioned. When a monoclonal antibody is used, a method wherein a hybridoma is prepared according to a conventional method, and secretion therefrom is harvested can be mentioned. As a method of producing an antibody fragment, a method wherein a fragment of a cloned antibody gene is expressed in a microorganism and the like is frequently used. The purity of the antibody, antibody fragment and the like is not particularly limited as long as it retains specific affinity for the protein. These antibodies and fragments thereof may be labeled with a fluorescent substance, an enzyme, a radioisotope and the like. Furthermore, commercially available ones may be used.

A substance having specific affinity for a gene encoding a marker protein is exemplified by oligo- or polynucleotide probe having specific affinity for the gene (hereinafter to be conveniently referred to simply as a probe), and an oligo- or polynucleotide primer pair having specific affinity for the gene (hereinafter to be conveniently referred to simply as a primer pair) can be mentioned. The "specific affinity" thereof refers to a property to hybridize only to the objective gene. Accordingly, it may be completely complementary to all or a part of the gene, or may include one to several mismatches as long as the above-mentioned property is satisfied. The probe and the primer pair are free of any particular limitation as long as they have specific affinity for the gene. For example, an oligo- or polynucleotide containing all or a part of the base sequence of the gene, a complementary sequence thereof, and the like can be mentioned, which are appropriately selected according to the form of the gene to be detected. The oligo- or polynucleotide is not particularly limited as to its origin as long as it has specific affinity for the gene. It may be a synthesized one or one obtained by cleaving out a necessary portion from the gene and purifying by a method generally employed. These oligo- and polynucleotides may be labeled with a fluorescence substance, an enzyme, a radioisotope and the like.

The separation or identification method of a pancreatic stem cell of the present invention is performed by analyzing 4, most preferably all the 5 markers, using at least 2, preferably 3, more preferably 4, most preferably all 5 substances, from 5 kinds of substances having specific affinity for each of the above-mentioned five marker proteins, or genes encoding the same. By such analysis, a pancreatic stem cell derived from a mammal can be obtained by separating a cell satisfying 4, most preferably 5, of the characteristics of [c-Met expressing, c-Kit non-expressing, CD45 non-expressing, TER119 non-expressing, Flk-1 non-expressing] (also described as [c-Met⁺, c-Kit⁻, CD45⁻, TER119⁻, Flk-1⁻]). Such stem cell shows a [c-Met⁺, c-Kit⁻, CD45⁻, TER119⁻, Flk-1⁻] marker. As used herein, by the "shows" is meant that it has a characteristic of "expressing" or "non-expressing" each marker protein or a gene encoding the same.

As a method of selecting and separating a cell showing each marker using each substance having specific affinity, a method generally employed in this field or a combination of such methods is used. For example, for an analysis of a marker at a protein level, particularly when a live cell needs to be recovered, a method, wherein a pigment for labeling the substance is appropriately selected and flowcytometry is utilized, is convenient. More preferably, a cell is separated using a fluorescence-activated cell sorter (FACS). By the use of such device, the objective cell can be automatically separated and recovered.

When recovery of a live cell is not particularly necessary, such as in the case of identification and the like, the cell may be crushed, extracted, and mRNA is recovered and subjected to Northern blot. Alternatively, a membrane protein may be extracted and subjected to Western blot.

As a method of separating a stem cell using gene expression as an index, for example, a step comprising expression of a GFP (green fluorescent protein) gene using a c-Met promoter, and separation by FACS can be utilized. This step (separation and identification of c-Met expressing cell) combined with a method using expression of a different marker as an index becomes a more effective means of separating and identifying a stem cell.

In the present invention, using the separation method and identification method of the present invention, a pancreatic stem cell of a mammal can be obtained or confirmed. Such stem cell can be differentiated into a cell having various physiological functions by induction of differentiation under suitable conditions (hereinafter a cell that has come to exert a physiological function by differentiation and induction in this manner is to be conveniently referred to as a functional cell). As used herein, by the "under suitable conditions" is meant being under culture conditions with the addition of cytokines such as a hepatocyte growth factor (HGF), an epidermal growth factor (EGF) and the like. For example, a pancreatic stem cell is known to differentiate into an α cell, which is a glucagon-producing cell, a β cell which is an insulin-producing cell, a γ cell, which is a pancreatic polypeptide-producing cell, a δ cell, which is a somatostatin-producing cell, an exocrine cell and the like, and such differentiation can be confirmed by examining the presence or absence of a physiologically active substance these cells express and secrete.

since a stem cell can be supplied stably, a cell differentiated from the stem cell can be also provided at a desired time in a desired amount. For example, it is expected that, by implanting a cell differentiated from a pancreatic stem cell into the body of a patient suffering from a pancreatic hypofunctional disease, the pancreatic function can be recovered and a therapeutic effect on a pancreatic hypofunctional disease can be afforded. As a pancreatic hypofunctional disease, diabetes, chronic pancreatitis, autoimmune pancreatitis, pancreatic functional disorder observed after removal of pancreas and the like can be mentioned. In addition, since a cell differentiated from a pancreatic stem cell expresses a liver· bile duct or stomach · intestine marker, it is expected that, by implanting a cell differentiated from a pancreatic stem cell into the body of a patient suffering from a liver· bile duct or stomach · intestinal hypofunctional disease, the liver · bile duct or stomach - intestinal function can be recovered, and a therapeutic effect on a liver- bile duct or stomach · intestinal hypofunctional disease can be afforded. As a liver- bile duct hypofunctional disease, acute hepatitis, chronic hepatitis, metabolic liver disease and the like can be mentioned, and as a stomach - intestinal hypofunctional disease, short bowel syndrome, inflammatory bowel disease, functional disorder of stomach observed after removal of the stomach and the like can be mentioned.

The specification also discloses a method of screening a substance that induces differentiation of a pancreatic stem cell, using the pancreatic stem cell of the present invention. This method comprises at least the following steps:
(1) a step of reacting a pancreatic stem cell with a test substance, and
(2) a step of determining an expression of a pancreatic marker in the cell after the reaction.

The pancreatic stem cell to be used in the screening method is not particularly limited as long as it differentiates into a functional cell of pancreas, which is preferably a pancreatic stem cell separated by the aforementioned method of the present invention. While the reaction between the stem cell and a test substance is appropriately set depending on the desired kind of differentiation, differentiation inducing conditions and the like, culture is generally performed at 35-40°C for several min to several dozen days, preferably at 36.5-37.5°C for 8 to 30 days. After the reaction with the test substance, the presence or absence and the state of the differentiation of the stem cell are examined.

Whether or not the stem cell has differentiated can be confirmed by determining the expression of the pancreatic marker. The marker is appropriately selected according to a desired functional cell. As used herein, the pancreatic marker is a protein that is expressed and produced (secreted) in a pancreas specific manner, particularly specifically to the functional cell of the pancreas, or a gene encoding the same. For example, the induction ability of differentiation into α cell can be confirmed by examining the production and secretion of glucagon and the induction ability of differentiation into β cell can be confirmed by examining the production and secretion of insulin.

The specification discloses a method of screening a substance that regulates the function of pancreas using a pancreatic stem cell or a functional cell differentiated from the stem cell by induction. This method comprises at least the following steps:.
(1) a step of reacting a pancreatic stem cell or a cell differentiated from the stem cell with a test substance, and
(2) a step of determining the expression of a pancreatic marker in the cell after the reaction.

The pancreatic stem cell and a functional cell differentiated from the stem cell to be used for this screening method are not particularly limited as long as they are a cell to be differentiated into a functional cell of the pancreas and a cell differentiated from the cell by induction, and are preferably a pancreatic stem cell separated by the aforementioned method of the present invention and a cell differentiated therefrom under suitable conditions (mentioned above). For example, the screening is performed concretely as follows. A test substance is added to a culture system of a pancreatic stem cell, and after culture for 8-30 days, expression of a gene or a protein (marker) in a functional cell differentiated by induction is analyzed, whereby the role of the test substance in the differentiation and proliferation of the pancreatic functional cell is analyzed (quantitative PCR, Northern blotting, immunostaining, ELISA etc.).

As a test substance to be the target in the screening method, various known and unknown substances are mentioned. Specifically, a known cytokine, an extracellular matrix, an inorganic compound, a culture supernatant.of a suitable cultured cell line, an unknown gene obtained from a suitable cDNA library, a recombinant protein of the gene and the like can be mentioned.

The specification also discloses a method of screening a substance that induces differentiation into the liver· bile duct or stomach · intestine using a pancreatic stem cell of the present invention. This method comprises at least the following steps:
(1) a step of reacting the pancreatic stem cell with a test substance, and
(2) a step of determining the expression of a liver- bile duct or stomach - intestine marker in the cell after the reaction.

While the reaction between the stem cell and a test substance is appropriately set depending on the desired kind of differentiation, differentiation-inducing conditions and the like, culture is generally performed at 35-40°C for several min to several dozen days, preferably at 36.5-37.5°C for 8 to 30 days. After the reaction with the test substance, the presence or absence and the state of the differentiation of the stem cell are examined.

Whether or not the stem cell has differentiated can be confirmed by determining the expression of the liver· bile duct or stomach · intestine marker. The marker is appropriately selected according to a desired functional cell. As used herein, the liver· bile duct or stomach - intestine marker is a protein that is expressed or produced (secreted) in a liver· bile duct or stomach · intestine specific manner, particularly specifically to the functional cell of the liver- bile duct or stomach - intestine, or a gene encoding the same. For example, the liver- bile duct cell marker includes albumin, α-fetoprotein, glucose-6-phosphatase, transthyretin, cytokeratin 19, cytokeratin 18, cytokeratin 8, biliary glycoprotein, and a gene encoding the same, and the stomach · intestine cell marker includes fabp-2, GIP, CCK, TPH, pepsinogen F, gastrin and a gene encoding the same.

The specification moreover discloses a method of screening a substance that regulates the function of the liver· bile duct or stomach · intestinal using the pancreatic stem cell of the present invention or a functional cell differentiated from the stem cell by induction. This method comprises at least the following steps:
(1) a step of reacting the pancreatic stem cell or a cell differentiated from the stem cell with a test substance, and
(2) a step of determining the expression of a liver · bile duct or stomach · intestine marker in the cell after the reaction.

For example, the screening is concretely performed as follows. A test substance is added to the culture system of a pancreatic stem cell, and after culture for 8-30 days, the expression of a gene or a protein (marker) in a functional cell differentiated from the stem cell by induction is analyzed; whereby the role of the test substance in the differentiation and proliferation of the pancreatic functional cell is analyzed (quantitative PCR, Northern blotting, immunostaining, ELISA etc.)..

As a test substance to be the target in the screening method, various known and unknown substances are mentioned. Specifically, a known cytokine, an extracellular matrix, an inorganic compound, a culture supernatant of a suitable cultured cell line, an unknown gene obtained from a suitable cDNA library, a recombinant protein of the gene and the like can be mentioned.

The pancreatic stem cell differentiation-inducing substance, a substance that induces differentiation into the liver· bile duct or stomach · intestine, a pancreatic function regulating substance, and a liver· bile duct or stomach· intestinal function regulating substance, which are obtained by such a screening method, are useful as therapeutic drugs of a pancreatic hypofunctional disease, liver· bile duct hypofunctional disease, and stomach - intestinal hypofunctional disease. Here, the pancreatic hypofunctional disease, liver· bile duct hypofunctional disease, and stomach · intestinal hypofunctional disease are as mentioned above.

A pharmaceutical agent containing the pancreatic stem cell or a cell differentiated from the stem cell is effective for the prophylaxis or treatment of pancreatic hypofunctional diseases, liver· bile duct hypofunctional diseases and stomach - intestinal hypofunctional diseases in various mammals such as human, monkey, mouse, rat, rabbit, pig, dog, horse, bovine and the like. While the dose is not particularly limited as long as it is an amount necessary for the treatment of the disease, it varies depending on the conditions (species, age, sex, body weight and the like) and symptoms of the subject in need of such treatment.

The pharmaceutical agent can contain a conventional pharmaceutically acceptable and substantially non-toxic carrier or excipient used for, for example, tablet, pellet, troche, capsule, suppository, cream, ointment, aerosol, powder preparation for inhalation, liquid, emulsion, suspension, other dosage forms suitable for use. In addition, an additive such as aid, stabilizer, thickener, colorant, flavor and the like can be added where necessary.

The pharmaceutical agent can be produced using a preparation technique known in the pertinent field. The pharmaceutical agent can be converted to a prodrug as necessary by a method known in this field.

### Examples

While the present invention is concretely explained in detail in the following by referring to Examples, the present invention is not limited in any way by these examples.

### Example 1

### Recovery of pancreatic cells

The pancreas was removed from C57BL/6 mouse neonate (one day after birth) under a stereo microscope, and placed in a Ca²⁺ free Hank's balanced salt solution containing 0.05% collagenase and 5 mmol/L CaCl₂ (pH 7.4). This was incubated at 37°C for 10 min and gently pipetting to isolate the cells, which cells were washed 3 times with a cell culture medium. After preparation, not less than 80% of cells were viable by detection with Trypan blue. As the cell culture medium, one having the following composition was independently prepared and used.

### (preparation of cell culture medium)

To a 1:1 mixture of DMEM and F-12 were prepared by adding fetal bovine serum (FBS, 10%) , γ-insulin (1 µg/mL), dexamethasone (1×10⁻⁷ M), nicotinamide (10 mM), L-glutamine (2 mM), β-mercaptoethanol (50 µM), HEPES (5 mM) and penicillin/streptomycin.

### flowcytometry

The prepared cells were first reacted with a biotinylated anti-CD45 monoclonal antibody (PharMingen, San Jose, CA), a biotinylated anti-TER119 monoclonal antibody (PharMingen, San Jose, CA), and an anti-c-Met monoclonal antibody (Upstate biotechnology, Lake Placid, NY) in ice water for 30 min. After the reaction, the cells were washed 3 times with a staining medium (PBS (phosphate-buffered saline) containing 3% FBS) and subsequently reacted with a FITC (fluorescein isothiocyanate)-labeled anti-mouse IgG2a monoclonal antibody (PharMingen, San Jose, CA), an APC (allophycocyanin)-labeled anti-c-Kit monoclonal antibody (PharMingen, San Jose, CA), and a Texasred-labeled streptavidin (Gibco BRL, GaithersBurg, MD) in ice water for 30 min. Finally, the cells were washed 3 times with a staining medium and suspended in a staining medium containing PI (propidium iodide) (5 µg/mL). These fluorescence-labeled cells were analyzed by FACS-vantage and separated into various fractions. The gate was set with the negative control as an index.

### Recovery of cells

CD45⁻, TER119⁻ cells were gated to remove CD45⁺ cell and TER119⁺ cell. By this operation, blood cells are removed. Then, CD45⁻, TER119⁻ cells were fractionated into c-Met⁺/c-Kit⁻, c-Met⁻/c-Kit⁺, c-Met⁺/c-Kit⁺ and c-Met⁻/c-Kit⁻ cell fractions and a cell recovery gate was set. Subsequently, the recovered cells were sown on a 35 mm diameter tissue culture dish at a concentration of 200 cells/cm² for low density culture. The residual erythrocytes, wreck of the cells, cell aggregation of 2 or more agglutinated cells and dead cells were removed by forward scattering, side scattering and PI. After the recovery, not less than 90% of the cells were viable by the detection with Trypan blue.

### In vitro colony assay

The cells recovered by FACS were cultured in the above-mentioned cell culture medium (37°C, 5% CO₂). After 24 hrs from the start of the culture, human recombinant hepatocyte growth factor (rHGF) (50 ng/mL; Sigma) and epidermal growth factor (EGF) (20 ng/mL; Sigma) were added. The number of colonies was counted at day 8 from the start of the culture.

### RT-PCR

The colonies were culture for 14 days and applied a cloning ring thereon, into which ISOGEN was added and gently pipetted, whereby RNA was obtained from a colony-forming cell. Subsequently, cDNA was prepared from the obtained RNA using reverse transcriptase (Super Script II). Then, using the prepared cDNA as a template and the following primer, PCR was conducted to try detection of the markers of various pancreatic functional cells.

The objective markers and their primers are shown in Table 1.

Each primer shown in Table 1 was ordered from Hokkaido System Science CO., LTD and synthesized for use.

The PCR cycle was 95°C (4 min) → [94°C (1 min) → 56°C (1 min) → 72°C (1 min) ]×45 cycles → 72°C (10 min). The nucleic acid produced by PCR was developed with 2% agarose gel and analyzed.

### Immunostaining

The colonies formed at day 14 of culture were washed 3 times with PBS, fixed with methanol (-20°C, 10 min) and washed 3 times with PBS containing Tween 20 (0.05%) (PBS-Tween 20). After blocking with 10% monkey serum, the colonies were reacted (4°C, 16 hrs) with a goat anti-insulin antibody or a mouse anti-glucagon antibody. After washing 3 times with PBS-Tween 20, blocking was performed again, and then the colonies were reacted (4°C, 3 hrs) respectively with an Alexa 488-labeled monkey anti-goat IgG antibody and a Cy3-labeled monkey anti-mouse IgG antibody. Finally, the colonies were washed 3 times with PBS-Tween 20, observed under Zeiss LSM510 laser scanning microscope and analyzed.

### Results

Pancreatic cells prepared from a mouse neonate were gated using FACS for viable cells (ca. 80%) and developed using antibodies against CD45 and TER119, respectively, to clarify the [CD45⁻, TER119⁻] cell fraction (ca. 40% of the whole), which was a non-blood cell fraction. Then, [CD45⁻, TER119⁻] cells were further developed with antibodies against c-Met (HGF receptor) and c-Kit (SCF receptor), respectively. The [c-Met⁺, c-Kit⁺, CD45⁻, TER119⁻] cells were about 0.01% of the whole, the [c-Met⁺, c-Kit⁻, CD45⁻, TER119⁻] cells were about 1.0% of the whole, the [c-Met⁻, c-Kit⁺, CD45⁻, TER119⁻] cells were about 0.8% of the whole, and the [c-Met⁻, c-Kit⁻, CD45⁻, TER119⁻] cells were about 36% of the whole. The cells contained in each fraction were recovered in a viable state and cultured (200 cells/cm²), and colonies were assayed in vitro. Some of the recovered cells showed high proliferation ability and they form a single cell-derived clonal colony. By observation at day 8 of culture, two kinds of colonies formed by morphologically distinct cells were mainly found. The colony formed by the epithelial cells was named an epithelial colony and the colony like a fibroblast was named a fibroblast-like colony. cDNA was separately prepared from the distinct colonies and the markers of various pancreatic functional cells were detected by PCR. As a result, pancreatic functional cell markers, such as insulin and glucagon, were detected only in epithelial colonies. Accordingly, it was strongly suggested that pancreatic stem/progenitor cells were contained in the epithelial colony-forming cells (epithelial colony-forming cell: ECFC). The ECFC was counted and, as a result, it was clarified that ECFC was confined to a [c-Met⁺, c-Kit⁻, CD45⁻, TER119⁻] cell fraction and present at high frequency (13.1-fold as compared to non-gated cells, and 9.45-fold as compared to [CD45⁻, TER119⁻] cell fraction).

Then, whether or not these ECFCs have multi-differentiation ability, which is among the characteristics of the pancreatic stem/progenitor cells, was analyzed by RT-PCR method and immunostain method. A total of 30 clonal colonies were analyzed by RT-PCR method, and as a result, many colonies in these colonies contained all or some of an α cell which is a glucagon-producing cell, a β cell which is an insulin-producing cell, a γ cell which is a pancreatic polypeptide-producing cell and a δ cell which is a somatostatin-producing cell, and a number of colonies containing a cell that expresses amylase or lipase, each of which is an exocrine cell marker, were also observed. Similarly, when the immunostain method was used, the co-presence of an α cell which is a glucagon-producing cell, and a β cell which is an insulin-producing cell, was observed in the same colony. From these results, it was clarified that a cell (ECFC) having a multi-differentiation ability, which is one of the important properties of a pancreatic stem/progenitor cell, is concentrated highly frequently in a [c-Met⁺, c-Kit⁻, CD45⁻, TER119⁻] cell fraction occupying only 1% of the mouse neonate pancreatic cells. It was simultaneously clarified that these cells can be separated and recovered alive using FACS.

### Example 2

### Recovery of pancreatic cells

Pancreatic cells were recovered in the same manner as in Example 1. The cell culture medium used was the same as the one used in Example 1.

### Flowcytometry

Conducted according to a similar method as in Example 1 except that, in addition to the various labeled antibodies and labeling reagents used in Example 1, PE (phycoerythrin)-labeled anti-Flk-1 monoclonal antibody (PharMingen, San Jose, CA) was also used.

### Recovery of cells

Conducted according to a similar method as in Example 1 except that the [c-Met⁺, c-Kit⁻, CD45⁻, TER119⁻] cells obtained in the same manner as in Example 1 were further fractionated into Flk-1 positive cells and negative cells.

### In vitro colony assay

Conducted according to a similar method as in Example 1.

### RT-PCR

Conducted according to a similar method as in Example 1.

### Immunostaining

Conducted according to a similar method as in Example 1.

### Results

The [c-Met⁺, c-Kit⁻, CD45⁻, TER119⁻] cells obtained in Example 1 were fractionated into Flk-1 positive and negative cells. The [c-Met⁺, c-Kit⁻, CD45⁻, TER119⁻, Flk-1⁻] cells were about 0.7% of the whole cells (all recovered pancreatic cells) and the [c-Met⁺, c-Kit⁻, CD45⁻, TER119⁻, Flk-1⁺] cells were about 0.3% of the whole cells. In the same manner as in Example 1, ECFC was counted. As a result, it was clarified that ECFC was further limited and contained at higher frequency in the [c-Met⁺, c-Kit⁻, CD45⁻, TER119⁻, Flk-1⁻] cell fraction, as compared to the [c-Met⁺, c-Kit⁻, CD45⁻, TER119⁻] cell fraction (19.7-fold as compared to non-gated cells, 14.1-fold as compared to [CD45⁻, TER119⁻] cell fraction).

Then, whether or not these ECFCs have multi-differentiation ability, which is among the characteristics of the pancreatic stem/progenitor cells, was analyzed by RT-PCR method and immunostaining method in the same manner as in Example 1. As a result, it was clarified that a cell (ECFC) having a multi-differentiation ability, which is one of the important properties of a pancreatic stem/progenitor cell, is concentrated at high frequently in a [c-Met⁺, c-Kit⁻, CD45⁻, TER119⁻, Flk-1⁻] cell fraction occupying only 0.7% of the mouse neonate pancreatic cells. It was simultaneously clarified that these cells could be separated and recovered alive using FACS.

### Example 3

### Recovery of pancreatic cells

Pancreatic cells were recovered in the same manner as in Example 1. The cell culture medium was the same as the one used in Example 1.

### Flowcytometry

Conducted according to a similar method as in Example 1 -except that, in addition to the various labeled antibodies and labeling reagents used in Example 1, PE (phycoerythrin)-labeled anti-Flk-1 monoclonal antibody (PharMingen, San Jose, CA) was also used.

### Recovery of cells

Conducted according to a similar method as in Example 1 except the [c-Met⁺, c-Kit⁻, CD45⁻, TER119⁻] cells obtained in the same manner as in Example 1 were further fractionated into Flk-1 positive cells and negative cells.

### In vitro culture

The cells recovered by FACS were cultured in the same cell culture medium as in Example 1 (37°C, 5% CO₂). At 24 hrs from the start of the culture, human recombinant hepatocyte growth factor (rHGF)(50 ng/mL; Sigma) and epidermal growth factor (EGF) (20 ng/mL; Sigma) were added. The cells were isolated by flowcytometry at day 30 from the start of the culture, and expression of the differentiation marker in the cells contained in the colony formed thereby was analyzed by RT-PCR.

### RT-PCR

Conducted according to a similar method as in Example 1.

### Results

The [c-Met⁺, c-Kit⁻, CD45⁻, TER119⁻, Flk-1⁻] cells were cultured for 30 days, isolated by flowcytometry and the expression of differentiation markers in the cells contained in the colony formed thereby was analyzed. As a result, the expression of albumin, α-fetoprotein, glucose-6-phosphatas, transthyretin, cytokeratin 19, cytokeratin 18, cytokeratin 8 and biliary glycoprotein as liver- bile duct cell markers, and the expression of fabp-2, GIP, CCK, TPH, pepsinogen F and gastrin as stomach - intestine cell markers was observed, in addition to various pancreatic cell markers.

**Table 1**

| marker | | primer |
|---|---|---|
| α cell specific marker | preproglucagon | 5'-ATTTACTTTGTGGCTGGATTG-3' (sequence No. 1) 5'-TGTCAGTGATCTTGGTTTGAA-3' (sequence No.2) |
| β cell specific marker | preproinsulin I | 5'-CTGTTGGTGCACTTCCTACC-3' (sequence No. 3) 5'-GCAGTAGTTCTCCAGCTGGT-3' (sequence No. 4) |
| | preproinsulin II | 5'-TCAAGCAGCACCTTTGTGGTT-3' (sequence No. 5) 5'-GTTGCAGTAGTTCTCCAGCTG-3' (sequence No. 6) |
| γ cell specific marker | pancreatic polypeptide | 5'-CTCCCTGTTTCTCGTATCCA-3' (sequence No. 7) 5'-AGAGCAGGGAATCAAGCCAA-3' (sequence No. 8) |
| δ cell specific marker | preprosomatostatin | 5'-CTCTGCATCGTCCTGGCTT-3' (sequence No. 9) 5'-CAGGATGTGAATGTCTTCCAG-3' (sequence No. 10) |
| exocrine cell specific marker | amylase 2 | 5'-AGTACCTGTGGAAGTTACCT-3' (sequence No. 11) 5'-ACACAAGGGCTCTGTCAGAA-3' (sequence No. 12) |
| | hormone-sensitive lipase | 5'-TCTTCTTCGAGGGTGATGAA-3' (sequence No. 13) 5'-TACCTTGCTGTCCTGTCCTT-3' (sequence No. 14) |
| Others | ipf1/pdx1 | 5'-TTACAAGCTCGCTGGGATCAC-3' (sequence No. 15) 5'-AGGTCACCGCACAATCTTGCT-3' (sequence No. 16) |
| | c-met | 5'-AGCCAGTAATGATCTCAATG-3' (sequence No. 17) 5'-TCAGGATAGGGGACAGGT-3' (sequence No. 18) |
| Positive control | hypoxanthine phosphoribosyl transferase (HPRT) | 5'-CTGTAATGATCAGTCAACGGC-3' (sequence No. 19) 5'-GGCCTATAGGCTCATAGTGCA-3' (sequence No. 20) |
| liver· bile duct cell marker | albumin | 5'-CATGACACCATGCCTGCTGAT-3' (sequence No. 21) 5'-CTCTGATCTTCAGGAAGTGTAC-3' (sequence No. 22) |
| | α-fetoprotein | 5'-ACTCACCCCAACCTTCCTGTC-3' (sequence No. 23) 5'-CAGCAGTGGCTGATACCAGAG-3' (sequence No. 24) |
| | glucose-6-phosphatase | 5'-AACCCATTGTGAGGCCAGAGG-3' (sequence No. 25) 5'-TACTCATTACACTAGTTGGTC-3' (sequence No. 26) |
| | transthyretin | 5'-TGGTATTTGTGTCTGAAGCTG-3' (sequence No. 27) 5'-TTAATAAGAATGCTTCACGGC-3' (sequence No. 28) |
| | cytokeratin 19 | 5'-GTCCTACAGATTGACAATGC-3' (sequence No. 29) 5'-CACGCTCTGGATCTGTGACAG-3' (sequence No. 30) |
| | cytokeratin 18 | 5'-GGACCTCAGCAAGATCATGGC-3' (sequence No. 31) 5'-CCACGATCTTACGGGTAGTTG-3' (sequence No. 32) |
| | cytokeratin 8 | 5'-AGTCTCAGATCTCAGACACG-3' (sequence No. 33) 5'-CCATAGGATGAACTCAGTCC-3' (sequence No. 34) |
| | biliary glycoprotein | 5'-GAACTAGACTCTGTCACCCTG-3' (sequence No. 35) 5'-GCCAGACTTCCTGGAATAGA-3' (sequence No. 36) |
| stomach· intestine cell marker | intestinal fatty acid binding protein; fabp-2 | 5'-ATTCGACGGCACGTGGAAAGT-3' (sequence No. 37) 5'-AAGAATCGCTTGGCCTCAACT-3' (sequence No. 38) |
| | gastric inhibitory peptide(GIP) | Jensen J., Pedersen E.E., Galante P., Hald J., Heller R.S., Ishibashi M., Kageyama R., Guillemot F., Serup P., and Madsen O.D. 2000. Control of endodermal endocrine development by Hes-1. Nat. Genet. 24: 36-44. |
| | cholecystokinin(CCK) | Jensen et al., *supra.* |
| | tryptophan hydroxylase (TPH) | Jensen et al., *supra.* |
| | pepsinogen F | 5'-ACCTAGACCTGGTCTACATTG-3' (sequence No. 39) 5'-AGTGAAGCTCTCCATGGTAGT-3' (sequence No. 40) |
| | gastrin | Jensen et al., *supra.* |

### Industrial Applicability

The pancreatic stem cell and various functional cells differentiated from the stem cell recover pancreas function by implanting the cells into the body of patients with pancreatic hypofunctional disease, such as diabetes and the like, and are expected to show a treatment effect on diabetes and the like. Moreover, a treatment effect on liver· bile duct or stomach - intestinal hypofunctional diseases can be also expected.

Furthermore, by the use of the stem cell and/or the functional cell, a substance that induces differentiation of pancreatic stem cell, a substance that induces differentiation into liver- bile duct or stomach - intestine, a pancreatic function regulating substance, and a liver- bile duct or stomach · intestinal function regulating substance can be screened. Such substance is promising as a therapeutic agent of pancreatic, liver· bile duct or stomach · intestinal hypofunctional diseases.

### Sequence Listing Free Text

Sequence No. 1: oligonucleotide designed to act as a PCR primer for detecting preproglucagon (α cell specific marker) gene.
Sequence No. 2: oligonucleotide designed to act as a PCR primer for detecting preproglucagon (α cell specific marker) gene.
Sequence No. 3: oligonucleotide designed to act as a PCR primer for detecting preproinsulin I (β cell specific marker) gene.
Sequence No. 4: oligonucleotide designed to act as a PCR primer for detecting preproinsulin I (β cell specific marker) gene.
Sequence No. 5: oligonucleotide designed to act as a PCR primer for detecting preproinsulin II (β cell specific marker) gene.
Sequence No. 6: oligonucleotide designed to act as a PCR primer for detecting preproinsulin II (β cell specific marker) gene.
Sequence No. 7: oligonucleotide designed to act as a PCR primer for detecting pancreatic polypeptide (γ cell specific marker) gene.
Sequence No. 8: oligonucleotide designed to act as a PCR primer for detecting pancreatic polypeptide (γ cell specific marker) gene.
Sequence No. 9: oligonucleotide designed to act as a PCR primer for detecting preprosomatostatin (δ cell specific marker) gene.
Sequence No. 10: oligonucleotide designed to act as a PCR primer for detecting preprosomatostatin (δ cell specific marker) gene.
Sequence No. 11: oligonucleotide designed to act as a PCR primer for detecting amylase 2 (exocrine cell specific marker) gene.
Sequence No. 12: oligonucleotide designed to act as a PCR primer for detecting amylase 2 (exocrine cell specific marker) gene.
Sequence No. 13: oligonucleotide designed to act as a PCR primer for detecting hormone-sensitive lipase (exocrine cell specific marker) gene.
Sequence No. 14: oligonucleotide designed to act as a PCR primer for detecting hormone-sensitive lipase (exocrine cell specific marker) gene.
Sequence No. 15: oligonucleotide designed to act as a PCR primer for detecting ipf1/pdx1 gene.
Sequence No. 16: oligonucleotide designed to act as a PCR primer for detecting ipf1/pdx1 gene.
Sequence No. 17: oligonucleotide designed to act as a PCR primer for detecting c-Met gene.
Sequence No. 18: oligonucleotide designed to act as a PCR primer for detecting c-Met gene.
Sequence No. 19: oligonucleotide designed to act as a PCR primer for detecting Hypoxanthine Phosphoribosyl Transferase (HPRT, positive control) gene.
Sequence No. 20: oligonucleotide designed to act as a PCR primer for detecting Hypoxanthine Phosphoribosyl Transferase (HPRT, positive control) gene.
Sequence No. 21: oligonucleotide designed to act as a PCR primer for detecting albumin (liver· bile duct cell marker) gene.
Sequence No. 22: oligonucleotide designed to act as a PCR primer for detecting albumin (liver· bile duct cell marker) gene.
Sequence No. 23: oligonucleotide designed to act as a PCR primer for detecting α-fetoprotein (liver· bile duct cell marker) gene.
Sequence No. 24: oligonucleotide designed to act as a PCR primer for detecting α-fetoprotein (liver· bile duct cell marker) gene.
Sequence No. 25: oligonucleotide designed to act as a PCR primer for detecting glucose-6-phosphatase (liver· bile duct cell marker) gene.
Sequence No. 26: oligonucleotide designed to act as a PCR primer for detecting glucose-6-phosphatase (liver· bile duct cell marker) gene.
Sequence No. 27: oligonucleotide designed to act as a PCR primer for detecting Transthyretin (liver· bile duct cell marker) gene.
Sequence No. 28: oligonucleotide designed to act as a PCR primer for detecting transthyretin (liver· bile duct cell marker) gene.
Sequence No. 29: oligonucleotide designed to act as a PCR primer for detecting cytokeratin 19 (liver· bile duct cell marker) gene.
Sequence No. 30: oligonucleotide designed to act as a PCR primer for detecting cytokeratin 19 (liver· bile duct cell marker) gene.
Sequence No. 31: oligonucleotide designed to act as a PCR primer for detecting cytokeratin 18 (liver· bile duct cell marker) gene.
Sequence No. 32: oligonucleotide designed to act as a PCR primer for detecting cytokeratin 18 (liver· bile duct cell marker) gene.
Sequence No. 33: oligonucleotide designed to act as a PCR primer for detecting cytokeratin 8 (liver· bile duct cell marker) gene.
Sequence No. 34: oligonucleotide designed to act as a PCR primer for detecting cytokeratin 8 (liver· bile duct cell marker) gene.
Sequence No. 35: oligonucleotide designed to act as a PCR primer for detecting biliary glycoprotein (liver· bile duct cell marker) gene.
Sequence No. 36: oligonucleotide designed to act as a PCR primer for detecting biliary glycoprotein (liver· bile duct cell marker) gene.
Sequence No. 37: oligonucleotide designed to act as a PCR primer for detecting intestinal fatty acid binding protein (stomach · intestine cell marker) gene.
Sequence No. 38: oligonucleotide designed to act as a PCR primer for detecting intestinal fatty acid binding protein (stomach · intestine cell marker) gene.
Sequence No. 39: oligonucleotide designed to act as a PCR primer for detecting pepsinogen F (stomach · intestine cell marker) gene.
Sequence No. 40: oligonucleotide designed to act as a PCR primer for detecting pepsinogen F (stomach · intestine cell marker) gene.

## Claims

1. A method of separating a pancreatic stem cell from the pancreas of a mammal using substances having specific affinity for four or five marker proteins or genes encoding the same selected from the group consisting of c-Met, c-Kit, CD45, TER119 and Flk-1.

2. The method of claim 1, which comprises a step of analysing the expression state of the four or five marker proteins or genes encoding the same.

3. The method of claim 1 or 2, wherein the substance having specific affinity is an antibody against the marker protein.

4. A method of identifying a pancreatic stem cell of a mammal using substances having specific affinity for four or five marker proteins or genes encoding the same selected from the group consisting of c-Met, c-Kit, CD45, TER119 and Flk-1.

5. The method of claim 4, which comprises a step of analysing the expression state of the four or five marker proteins or genes encoding the same.

6. The method of claim 4 or 5, wherein the substance having specific affinity is an antibody against the marker protein.

## Patentansprüche

1. Verfahren zum Trennen einer pankreatischen Stammzelle von der Pankreas eines Säugers unter Verwendung von Substanzen mit spezifischer Affinität für vier oder fünf Markerproteine oder Gene, die diese codieren, ausgewählt aus der Gruppe bestehend aus c-Met, c-Kit, CD45, TER119 und Flk-1.

2. Verfahren gemäß Anspruch 1, das einen Schritt des Analysierens des Expressionszustands der vier oder fünf Markerproteine oder Gene, die diese codieren, umfaßt.

3. Verfahren gemäß Anspruch 1 oder 2, worin die Substanz mit spezifischer Affinität ein Antikörper ist, der gegen das Markerprotein gerichtet ist.

4. Verfahren zum Identifizieren einer pankreatischen Stammzelle eines Säugers unter Verwendung von Substanzen mit spezifischer Affinität für vier oder fünf Markerproteine oder Gene, die diese codieren, ausgewählt aus der Gruppe bestehend aus c-Met, c-Kit, CD45, TER119 und Flk-1.

5. Verfahren gemäß Anspruch 4, das einen Schritt des Analysierens des Expressionszustands der vier oder fünf Markerproteine oder Gene, die diese codieren, umfaßt.

6. Verfahren gemäß Anspruch 4 oder 5, worin die Substanz mit spezifischer Affinität ein Antikörper ist, der gegen das Markerprotein gerichtet ist.

## Revendications

1. Procédé de séparation d'une cellule souche pancréatique à partir du pancréas d'un mammifère au moyen de substances présentant une affinité spécifique pour quatre ou cinq protéines marqueurs ou pour les gènes codant pour celles-ci, choisi(e)s dans le groupe constitué de c-Met, c-Kit, CD45, TER119 et Flk-1.

2. Procédé selon la revendication 1, comprenant une étape d'analyse de la situation, en matière d'expression, des quatre ou cinq protéines marqueurs ou des gènes codant pour celles-ci.

3. Procédé selon la revendication 1 ou 2, dans lequel la substance présentant une affinité spécifique est un anticorps dirigé contre la protéine marqueur.

4. Procédé d'identification d'une cellule souche pancréatique d'un mammifère au moyen de substances présentant une affinité spécifique pour quatre ou cinq protéines marqueurs ou pour les gènes codant pour celles-ci, choisi(e)s dans le groupe constitué de c-Met, c-Kit, CD45, TER119 et Flk-1.

5. Procédé selon la revendication 4, comprenant une étape d'analyse de la situation, en matière d'expression, des quatre ou cinq protéines marqueurs ou des gènes codant pour celles-ci.

6. Procédé selon la revendication 4 ou 5, dans lequel la substance présentant une affinité spécifique est un anticorps dirigé contre la protéine marqueur.


### SEQUENCE.LISTING

<110> AJINOMOTO CO., INC.
<120> Stem cell and separating method thereof
<130> 09474
<150> JP 2001-126315
   <151> 2001-04-24
<160> 40
<170> PatentIn version 3.1
<210> 1
   <211> 21
   <212> DNA
   <213> Artificial Sequence
   <220>
   <223> Oligonucleotide designed to act as PCR primer for detection of prepr oglucagon (alpha cell specific marker) gene
   <400> 1
   atttactttg tggctggatt g 21
<210> 2
   <211> 21
   <212> DNA
   <213> Artificial Sequence
   <220>
   <223> Oligonucleotide designed to act as PCR primer for detection of prepr oglucagon (alpha cell specific marker) gene
   <400> 2
   tgtcagtgat cttggtttga a 21
<210> 3
   <211> 20
   <212> DNA
   <213> Artificial Sequence
   <220>
   <223> Oligonucleotide designed to act as PCR primer for detection of prepr oinsulin I (beta cell specific marker) gene
   <400> 3
   ctgttggtgc acttcctacc 20
<210> 4
   <211> 20
   <212> DNA
   <213> Artificial Sequence
   <220>
   <223> Oligonucleotide designed to act as PCR primer for detection of prepr oinsulin I (beta cell specific marker) gene
   <400> 4
   gcagtagttc tccagctggt 20
<210> 5
   <211> 21
   <212> DNA
   <213> Artificial Sequence
   <220>
   <223> Oligonucleotide designed to act as PCR primer for detection of prepr oinsulin II (beta cell specific marker) gene
   <400> 5
   tcaagcagca cctttgtggt t 21
<210> 6
   <211> 21
   <212> DNA
   <213> Artificial Sequence
   <220>
   <223> Oligonucleotide designed to act as PCR primer for detection of prepr oinsulin II (beta cell specific marker) gene
   <400> 6
   gttgcagtag ttctccagct g 21
<210> 7
   <211> 20
   <212> DNA
   <213> Artificial Sequence
   <220>
   <223> Oligonucleotide designed to act as PCR primer for detection of pancr eatic polypetide (gamma cell specific marker) gene
   <400> 7
   ctccctgttt ctcgtatcca 20
<210> 8
   <211> 20
   <212> DNA
   <213> Artificial Sequence
   <220>
   <223> Oligonucleotide designed to act as PCR primer for detection of pancr eatic polypetide (gamma cell specific marker) gene
   <400> 8
   agagcaggga atcaagccaa 20
<210> 9
   <211> 19
   <212> DNA
   <213> Artificial Sequence
   <220>
   <223> Oligonucleotide designed to act as PCR primer for detection of prepr osomatostatin (delta cell specific marker) gene
   <400> 9
   ctctgcatcg tcctggctt 19
<210> 10
   <211> 21
   <212> DNA
   <213> Artificial Sequence
   <220>
   <223> Oligonucleotide designed to act as PCR primer for detection of prepr osomatostatin (delta cell specific marker) gene
   <400> 10
   caggatgtga atgtcttcca g 21
<210> 11
   <211> 20
   <212> DNA
   <213> Artificial Sequence
   <220>
   <223> Oligonucleotide designed to act as PCR primer for detection of amyla se 2 (exocrine cell specific marker) gene
   <400> 11
   agtacctgtg gaagttacct 20
<210> 12
   <211> 20
   <212> DNA
   <213> Artificial Sequence
   <220>
   <223> Oligonucleotide designed to act as PCR primer for detection of amyla se 2 (exocrine cell specific marker) gene
   <400> 12
   acacaagggc tctgtcagaa 20
<210> 13
   <211> 20
   <212> DNA
   <213> Artificial Sequence
   <220>
   <223> Oligonucleotide designed to act as PCR primer for detection of hormo ne-sensitive lipase (exocrine cell specific marker) gene
   <400> 13
   tcttcttcga gggtgatgaa 20
<210> 14
   <211> 20
   <212> DNA
   <213> Artificial Sequence
   <220>
   <223> Oligonucleotide designed to act as PCR primer for detection of hormo ne-sensitive lipase (exocrine cell specific marker) gene
   <400> 14
   taccttgctg tcctgtcctt 20
<210> 15
   <211> 21
   <212> DNA
   <213> Artificial Sequence
   <220>
   <223> Oligonucleotide designed to act as PCR primer for detection of ipf1/ pdx1 gene
   <400> 15
   ttacaagctc gctgggatca c 21
<210> 16
   <211> 21
   <212> DNA
   <213> Artificial Sequence
   <220>
   <223> Oligonucleotide designed to act as PCR primer for detection of ipf1/ pdx1 gene
   <400> 16
   aggtcaccgc acaatcttgc t 21
<210> 17
   <211> 20
   <212> DNA
   <213> Artificial Sequence
   <220>
   <223> Oligonucleotide designed to act as PCR primer for detection of c-met gene
   <400> 17
   agccagtaat gatctcaatg 20
<210> 18
   <211> 18
   <212> DNA
   <213> Artificial Sequence
   <220>
   <223> Oligonucleotide designed to act as PCR primer for detection of c-met gene
   <400> 18
   tcaggatagg ggacaggt 18
<210> 19
   <211> 21
   <212> DNA
   <213> Artificial Sequence
   <220>
   <223> Oligonucleotide designed to act as PCR primer for detection of hypox anthine phosphoribosyltransferase (HPRT, positive control) gene
   <400> 19
   ctgtaatgat cagtcaacgg c 21
<210> 20
   <211> 21
   <212> DNA
   <213> Artificial Sequence
   <220>
   <223> Oligonucleotide designed to act as PCR primer for detection of hypox anthine phosphoribosyltransferase (HPRT, positive control) gene
   <400> 20
   ggcctatagg ctcatagtgc a 21
<210> 21
   <211> 21
   <212> DNA
   <213> Artificial Sequence
   <220>
   <223> Oligonucleotide designed to act as PCR primer for detection of album in (hepatocyte/biliary cell marker) gene
   <400> 21
   catgacacca tgcctgctga t 21
<210> 22
   <211> 22
   <212> DNA
   <213> Artificial Sequence
   <220>
   <223> Oligonucleotide designed to act as PCR primer for detection of album in (hepatocyte/biliary cell marker) gene
   <400> 22
   ctctgatctt caggaagtgt ac 22
<210> 23
   <211> 21
   <212> DNA
   <213> Artificial Sequence
   <220>
   <223> Oligonucleotide designed to act as PCR primer for detection of alpha -fetoprotein (hepatocyte/biliary cell marker) gene
   <400> 23
   actcacccca accttcctgt c 21
   <210> 24
   <211> 21
   <212> DNA
   <213> Artificial Sequence
   <220>
   <223> Oligonucleotide designed to act as PCR primer for detection of alpha -fetoprotein (hepatocyte/biliary cell marker) gene
   <400> 24
   cagcagtggc tgataccaga g 21
<210> 25
   <211> 21
   <212> DNA
   <213> Artificial Sequence
   <220>
   <223> Oligonucleotide designed to act as PCR primer for detection of gluco se-6-phosphatase (hepatocyte/biliary cell marker) gene
   <400> 25
   aacccattgt gaggccagag g 21
<210> 26
   <211> 21
   <212> DNA
   <213> Artificial Sequence
   <220>
   <223> Oligonucleotide designed to act as PCR primer for detection of gluco se-6-phosphatase (hepatocyte/biliary cell marker) gene
   <400> 26
   tactcattac actagttggt c 21
<210> 27
   <211> 21
   <212> DNA
   <213> Artificial Sequence
   <220>
   <223> Oligonucleotide designed to act as PCR primer for detection of trans thyretin (hepatocyte/biliary cell marker) gene
   <400> 27
   tggtatttgt gtctgaagct g 21
<210> 28
   <211> 21
   <212> DNA
   <213> Artificial Sequence
   <220>
   <223> Oligonucleotide designed to act as PCR primer for detection of trans thyretin (hepatocyte/biliary cell marker) gene
   <400> 28
   ttaataagaa tgcttcacgg c 21
<210> 29
   <211> 20
   <212> DNA
   <213> Artificial Sequence
   <220>
   <223> Oligonucleotide designed to act as PCR primer for detection of cytok eratin 19 (hepatocyte/biliary cell marker) gene
   <400> 29
   gtcctacaga ttgacaatgc 20
<210> 30
   <211> 21
   <212> DNA
   <213> Artificial Sequence
   <220>
   <223> Oligonucleotide designed to act as PCR primer for detection of cytok eratin 19 (hepatocyte/biliary cell marker) gene
   <400> 30
   cacgctctgg atctgtgaca g 21
<210> 31
   <211> 21
   <212> DNA
   <213> Artificial Sequence
   <220>
   <223> Oligonucleotide designed to act as PCR primer for detection of cytok eratin 18 (hepatocyte/biliary cell marker) gene
   <400> 31
   ggacctcagc aagatcatgg c 21
   <210> 32
   <211> 21
   <212> DNA
   <213> Artificial Sequence
   <220>
   <223> Oligonucleotide designed to act as PCR primer for detection of cytok eratin 18 (hepatocyte/biliary cell marker) gene
   <400> 32
   ccacgatctt acgggtagtt g 21
<210> 33
   <211> 20
   <212> DNA
   <213> Artificial Sequence
   <220>
   <223> Oligonucleotide designed to act as PCR primer for detection of cytok eratin 8 (hepatocyte/biliary cell marker) gene
   <400> 33
   agtctcagat ctcagacacg 20
<210> 34
   <211> 20
   <212> DNA
   <213> Artificial Sequence
   <220>
   <223> Oligonucleotide designed to act as PCR primer for detection of cytok eratin 8 (hepatocyte/biliary cell marker) gene
   <400> 34
   ccataggatg aactcagtcc 20
<210> 35
   <211> 21
   <212> DNA
   <213> Artificial Sequence
   <220>
   <223> Oligonucleotide designed to act as PCR primer for detection of bilia ry glycoprotein (hepatocyte/biliary cell marker) gene
   <400> 35
   gaactagact ctgtcaccct g 21
<210> 36
   <211> 20
   <212> DNA
   <213> Artificial Sequence
   <220>
   <223> Oligonucleotide designed to act as PCR primer for detection of bilia ry glycoprotein (hepatocyte/biliary cell marker) gene
   <400> 36
   gccagacttc ctggaataga 20
<210> 37
   <211> 21
   <212> DNA
   <213> Artificial Sequence
   <220>
   <223> Oligonucleotide designed to act as PCR primer for detection of intes tinal fatty acid binding protein (fabp-2; gastric cell/enterocyte marker) g ene
   <400> 37
   attcgacggc acgtggaaag t 21
<210> 38
   <211> 21
   <212> DNA
   <213> Artificial Sequence
   <220>
   <223> Oligonucleotide designed to act as PCR primer for detection of intes tinal fatty acid binding protein (fabp-2; gastric cell/enterocyte marker) g ene
   <400> 38
   aagaatcgct tggcctcaac t 21
<210> 39
   <211> 21
   <212> DNA
   <213> Artificial Sequence
   <220>
   <223> Oligonucleotide designed to act as PCR primer for detection of pepsi nogen F (gastric cell/enterocyte marker) gene
   <400> 39
   acctagacct ggtctacatt g 21
   <210> 40
   <211> 21
   <212> DNA
   <213> Artificial Sequence
   <220>
   <223> Oligonucleotide designed to act as PCR primer for detection of pepsi nogen F (gastric cell/enterocyte marker) gene
   <400> 40
   agtgaagctc tccatggtag t 21